# EUROPÄISCHE PATENTANMELDUNG

(11) **EP 1 285 964 A2**
(43) Veröffentlichungstag der Anmeldung: **26.02.2003**
(21) Anmeldenummer: 02090246.6
(22) Anmeldetag: 12.07.2002
(51) Int. Cl.: C12N 15/09, C07K 14/47, C07K 16/18, C12Q 1/68, C12N 15/11, G01N 33/50

(54) **Humane Mater Proteine**

(30) Priorität: 10.08.2001 DE 10139874
(71) Anmelder: SCHERING AKTIENGESELLSCHAFT, 13353 Berlin (DE)
(72) Erfinder: Weiss, Bertram, 14163 Berlin (DE); Lessl, Monika, 16548 Gienicke-Nordbahn (DE); Peters-Kottig, Michaele, 10437 Berlin (DE); Beckmann, Georg, 10439 Berlin (DE)

(57) **Zusammenfassung**

Es werden zwei humane MATER-Proteine sowie deren Verwendung für Fertilitätsstörungen, Therapie und Diagnose beschrieben.

## Beschreibung

Die Erfindung betrifft Mater und dessen Verwendung als Arzneimittel für die Beeinflußung von apoptotischen Prozessen, die eine grundlegende Rolle spielen bei der Fertilitätskontrolle.

Zur Kontrolle der Fertilität ist die "klassische Pille" als weibliches Kontrazeptivum, das am häufigste eingesetzte Mittel der Wahl. Ihre regelmäßige Einnahme führt zur Ovulationshemmung bei der Frau. Das Prinzip dieser Methode ist, dass es durch die Einnahme der Pille zu einer Unterdrückung der endogenen Steroidhormonproduktion im Ovar und somit zu einer Ovulationshemmung kommt. Ein Nachteil ist, dass ein natürlicher Zyklus somit bei der Frau nicht mehr vorhanden ist. Zudem können in Zusammenhang mit der Einnahme der Pille bei Patientinnen mit Risikopotential Nebenwirkungen wie beispielsweise Brustspannungen, Gewichtszunahmen etc. auftreten.

Durch zahlreiche Untersuchungen ist belegt, dass bei der Frau mit zunehmendem Alter die Fertilität abnimmt. Dies ist unter anderem auf eine schlechtere Qualität der Eizelle, eine erhöhte Abortrate, verstärkte Exposition mit Infektionskeimen (z.B. Chlamydien oder Gonokokken) zurückzuführen. Da sich in den Industrieländern jedoch das Alter der Erstgebährenden immer weiter nach hinten verschiebt, ist es notwendig, Möglichkeiten zur Verbesserung der Fertilität zu finden. Dieses trifft für die Frau und auch für den Mann zu. Für Paare mit Fertilitätsstörungen stehen heute die Techniken der assistierten Reproduktion zur Verfügung (*in vitro* Fertilisation (IVF); Gamete intrafallopian transfer (GIFT), Intrauterine Insemination). Diese Methoden sind jedoch invasiv und in den meisten Fällen mit einer vorherigen Stimulation der Follikelreifung bei der Frau durch Proteohormone (Follikel-stimulierendes Hormon/FSH) verbunden. Diese kann zu unangenehmen Nebenwirkungen wie Kopfschmerzen oder Schmerzen im Abdomen, im schlimmsten Fall jedoch auch zum sogenannten ovariellen Hyperstimulationssyndrom führen.

Es besteht die dringende Notwendigkeit neue Substanzen und Mittel zur Fertilitätskontrolle, das heißt sowohl zur Fertilitätsförderung als auch zur Fertilitätshemmung, bereitzustellen.

Bei Mäusen wurde gezeigt, dass weibliche Tiere ohne ein OP (Ooplasm-specific protein) -1 Gen infertil sind (Tong und Nelson, 1999, Endocrinology 140, 3720 - 3726 und Tong *et al.*, Nature Genetics, 2000, 26, 267 - 267), während die Fertilität der männlichen Tiere unverändert bleibt. Es konnte gezeigt werden, dass die Infertilität durch eine Blockade der Entwicklung der befruchteten Eizelle nach dem Zwei-Zell-Stadium zustande kommt. Der Zyklus der Mäuse ist normal, Tiere ovulieren spontan nach Stimulation mit Gonadotropin. Befruchtete Zellen dieser transgenen Tiere jedoch verbleiben im Zwei-Zell-Stadium ohne weitere Entwicklung bzw. degenerieren ca 3 Tage nach Fertilisation. Das OP-1 wird auch als MATER (matemal-antigen-that-embryos-require) bezeichnet.

In einem Maus-Modell der Autoimmun-Oophoritis konnten Antikörper gegen Maus-MATER-Protein nachgewiesen werden. Dieses Modell hat viele Gemeinsamkeiten mit dem humanen Krankheitsbild des ,autoimmune-premature ovarian-failure, so dass die Möglichkeit besteht, dass ein putatives humanes MATER-Protein eine ähnlich bedeutende Rolle spielt bei der Regulation der Fertilität wie das Maus-MATER. Allerdings ist bisher nicht bekannt, ob ein humanes Homolog zum Maus-MATER Protein existiert.

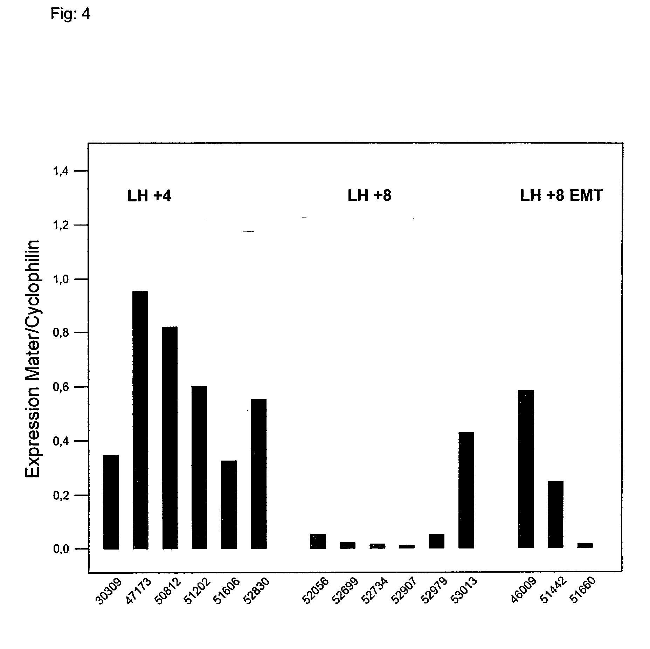

In der vorliegenden Erfindung konnte gezeigt werden, dass humanes MATER stark in der Gebährmutter (Uterus) exprimiert wird. Dabei zeigt die Expression in der inneren Schicht des Uterus, dem Endometrium, eine deutliche Regulation in Abhängigkeit vom Zyklus der Frau. Die Expression von Mater ist im sogenannten Implantationsfenster des Zyklus (ca. 8 Tage nach dem Anstieg des luteinisierenden Hormons LH, das die Ovulation auslöst) deutlich erniedrigt im Vergleich zur Phase direkt nach der Ovulation (siehe Figur 4). Mittels quantitativer Bestimmungen wurde die mRNA aus dem humanen Endometrium dreier verschiedener klinischer Studiengruppen miteinander verglichen. Das Ergebnis dieser Studien ergab, dass die mRNA von MATER im Vergleich zur Kontrollgruppe (vor Öffnung des Implantationsfensters) in der Gruppe LH +8 (Zeit des offenen Implantationsfensters) bei gesunden Frauen stark erniedrigt ist. In der Gruppe LH +8/EMT (Endometriose-Patientinnen) konnten in 2 von 3 Patientinnen erhöhte Menge an MATER-mRNA festgestellt werden. Eine Ursache der Infertilität, wie sie bei Endometriose-Patientinnen beobachtet wird, kann auf der erhöhten MATER-Expression beruhen, da im Implantationsfenster, also zum Zeitpunkt der fertilen Phase, die MATER-Expression im Endometrium von fertilen Frauen erniedrigt ist.

Ein humanes MATER-Protein stellt daher eine geeignete Zielsubstanz dar, um neue Mittel zur Fertilitätskontrolle zu identifizieren.

Die vorliegende Erfindung stellt eine Nukleinsäure bereit umfassend
a. die in Seq ID NO 1 oder Seq ID NO 3 dargestellte Nukleotidsequenz,
b. eine einer Sequenz aus a. im Rahmen der Degeneration des genetischen Codes entsprechende Nukleotidsequenz oder
c. eine mit den Sequenzen aus a. oder b. unter stringenten Bedingungen hybridisierende Nukleotidsequenz mit der Funktion eines humanen MATER-Proteins.

Der Begriff "Hybridisierung unter stringenten Bedingungen" gemäß der vorliegenden Erfindung wird bei Sambrook et al. (Molecular Cloning, A Laboratory Manual, Cold Spring Harbor Laboratory Press, 1989) definiert. Eine stringente Hybridisierung liegt beispielsweise vor, wenn nach dem Waschen für 1 h mit 1 x SSC und 0,1%SDS bei 50°C, vorzugsweise bei 55°C, besonders bevorzugt bei 62°C und am meisten bevorzugt bei 68°C, insbesondere für 1h in 0,2 x SSC und 0,1 % SDS bei 55°C, vorzugsweise bei 62°C und am meisten bevorzugt bei 68°C noch ein Hybridisierungssignal beobachtet wird. Die Nukleinsäuren, die unter diesen Bedingungen mit der in Seq ID NO 1 und/oder 3 gezeigten Nukleinsäure oder einer dieser Sequenz im Rahmen der Degeneration des genetischen Codes entsprechende Nukleotidsequenz hybridisiert, sind auch Gegenstand der vorliegenden Erfindung.

Nukleinsäuren können einzel- oder doppelsträngige DNA, z.B. cDNA, oder RNA, z.B. mRNA, cRNA, pre-mRNA darstellen.

Die in Seq ID NO1 und Seq ID NO 3 dargestellten Nukleinsäuren kodieren für ein humanes MATER Protein. Sie stellen Splice-Varianten desselben Gens dar. Bei der in Seq ID NO 3 dargestellten Sequenz fehlt das Exon 4.

Bevorzugt ist die Nukleinsäure, die einen Protein kodierenden Abschnitt der in Seq ID NO 1 oder Seq ID No 3 dargestellten Nukleinsäuresequenz umfaßt. Ein Protein kodierender Abschnitt der in Seq ID NO 1 dargestellten Sequenz liegt im Bereich von Nukleotid 1 bis 3489 und der in Seq ID NO3 dargestellten Sequenz im Bereich von Nukleotid 1 bis 3432.

Gegenstand der Erfindung ist ferner eine Nukleinsäure, die für ein Polypeptid mit der in Seq ID NO 2 oder Seq ID NO 4 dargestellten Aminosäuresequenz kodiert.

Die erfindungsgemäße Nukleinsäure ist aus Säugern, z.B. humanen Zellen oder aus einer cDNA-Bibliothek oder einer genomischen Bibliothek, die z.B. aus menschlichen Zellen gewonnen wird, erhältlich. Sie kann nach bekannten Techniken unter Verwendung kurzer Abschnitte der in Seq ID NO 1 oder Seq ID NO 3 gezeigten Nukleinsäureseqenz als Hybridisierungssonden oder Amplifikationsprimer isoliert werden.

Die Erfindung betrifft weiterhin Polypeptide, die von einer erfindungsgemäßen Nukleinsäure kodiert werden. Diese Polypeptide haben die Funktion eines humanen MATER Proteins. Die Funktion des MATER-Proteins ist die einer NTPase, die in Zusammenhang steht mit der Apoptose. Durch Fehlfunktionen im MATER-Protein kommt es zu einem Arrest der Entwicklung der sich im Zwei-Zell-Stadium befindenden befruchteten Eizelle. Die Zellen unterlaufen Apoptose, eine weitere Entwicklung ist nicht mehr möglich.

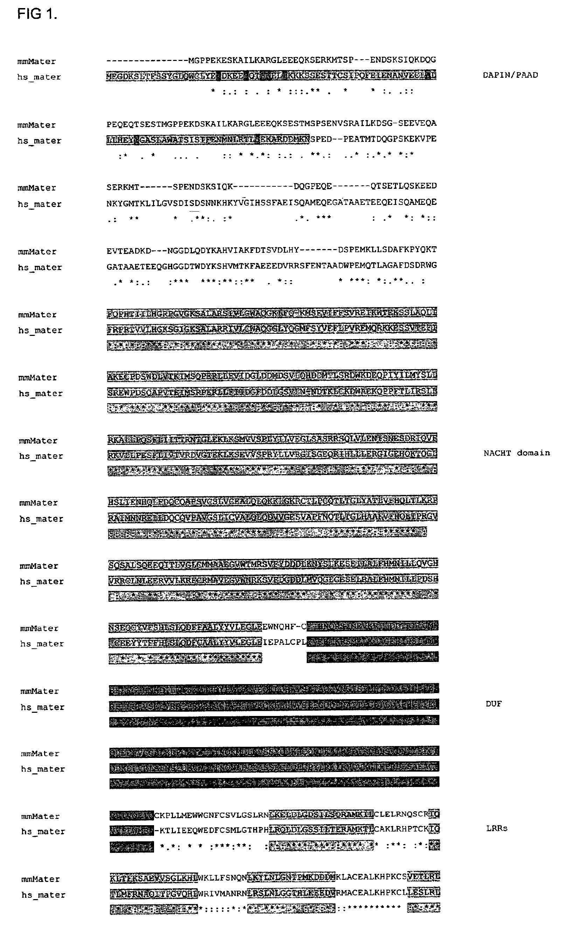

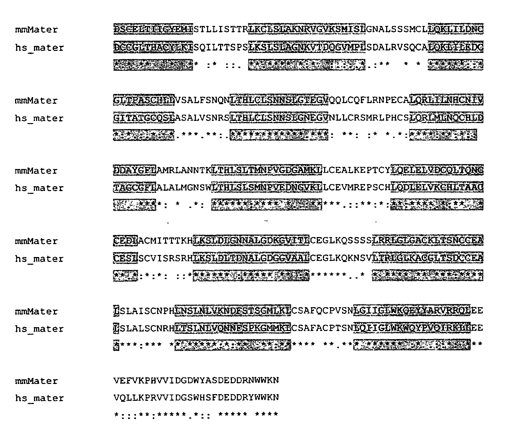

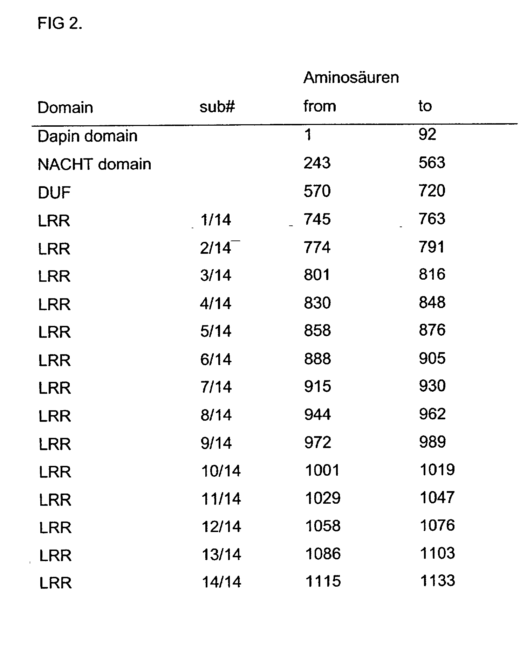

Weiterhin Gegenstand der Erfindung ist ein Polypeptid, das die in Seq ID NO 2 oder Seq ID NO 4 dargestellte Aminosäuresequenz umfasst.
Das erfindungsgemäße Polypeptid kann ein rekombinantes Polypeptid, ein natürliches, isoliertes Polypeptid oder ein synthetisches Polypeptid sein.
Das erfindungsgemäße Polypeptid enthält verschiedene Domänen: eine Dapin-(Domain in apoptosis and interferon response), eine NACHT (NAIP, CIIA, HET- and TP-1) und eine DUF( Domain of unknown function) - Domäne. Diese Domänen werden in Zusammenhang mit Apoptose gebracht (Staub E. *et al*., TIBS, 2001, 26 (2), 83 - 85, Koonin EV, Aravind L., TIBS, 2000, 25 (5), 223 - 224). Es gibt Hinweise darauf, dass Mitglieder der NACHT-Familie NTPasen beinhalten, die anti-apoptotische Wirkung haben. Die Dapin-Domänen, die verantwortlich sind für die Ausbildung von Homo-oder Hetero-Dimeren, wurden bisher bei Proteinen beschrieben, die in Apoptose oder inflammatorische Prozesse involviert sind. MATER wirkt antiapoptotisch, beim Fehlen bzw. bei Fehlfunktionen des MATER-Proteins gehen die Zellen in die Apoptose, eine weitere Entwicklung des Zwei-Zell-Stadiums ist nicht mehr möglich. Außerdem enthält MATER 14 sogenannte "Leucin-rich-repeats" (LRR, Kajava AV, 1998, J. Mol. Biol. 227, 519 - 527), die für die Protein-Protein-Interaktionen verantwortlich sind. Obwohl die Homologie zwischen der humanen und Maus-Sequenz nur nur 52% beträgt, zeigen Maus-Mater -und humanes Mater-Polypeptid in den Bereichen aller Domänen hohe Sequenzhomologie, mit Ausnahme der Dapin-Domäne (siehe Abbildungen 1 und 2).

Die mRNA des erfindungsgemäßen Polypeptids nach Seq ID NO 2 oder Seq ID NO 4 wird vorwiegend in Ovarien, in Testes und in der Plazenta transkribiert.

Das erfindungsgemäße Polypeptid oder Teilbereiche davon (Peptide) können zur Herstellung von Antikörpern verwendet werden. Zur Herstellung polyklonaler Antikörper können die Polypeptide oder Peptide z.B. an KLH (Keyhole Limpet Hemocyanin) gebunden werden und Tieren, z.B. Kaninchen, gespritzt werden. Sie können auch zur Herstellung monoklonaler Antikörper verwendet werden. Zur Antikörperherstellung kann ein erfindungsgemäßes Polypeptid oder Peptid oder eine Mischung mehrerer erfindungsgemäßer Peptide verwendet werden. Die Herstellung der Antikörper erfolgt dabei nach Standardverfahren wie sie z.B. in Kohler, G. und Milstein, C., Nature 1975, 256, 495 - 497 und Nelson, P. N. *et al.*, Mol. Pathol. 2000, 53, 111 - 117 beschrieben sind.

Gegenstand der Erfindung sind auch die Antikörper, die gegen ein erfindungsgemäßes Polypeptid gerichtet sind.
Die erfindungsgemäßen Antikörper können zur Detektion der erfindungsgemäßen Polypeptide verwendet werden. Dies kann z.B. durch Immunhistochemie erfolgen. Die erfindungsgemäßen Antikörper können auch in anderen Immuntests wie z.B. einem ELISA (enzyme linked immunosorbent assay) oder in Radioimmunotests eingesetzt werden. So kann die Konzentration an erfindungsgemäßen Polypeptid in Gewebe- oder Zellextrakten nachgewiesen werden.

Der Nachweis der Expression des erfindungsgemäßen Polypeptids kann auch über den Nachweis von mRNA in den Zellen erfolgen. Gegenstand der Erfindung ist daher auch die Verwendung einer Sonde mit Nukleinsäuresequenzen, die komplementär zu den Nukleinsäuresequenzen sind, die für die erfindungsgemäßen Peptide kodieren, zur Herstellung eines Reagenz zum Nachweis der Gegenwart von erfindungsgemäßer mRNA in Zellen. Eine Sonde ist ein kurzes DNA-Stück mit mindestens 14 Nukleotiden. Die erfindungsgemäßen Sonden können z.B. in einer Northern Blot Analyse verwendet werden. Diese Methode ist z.B. in Sambrook, J. *et al*., 1989, Cold Spring Harbor Laboratory Press, beschrieben. Andere Methoden zum Nachweis der RNA sind *in situ* Hybridisierung, RNAse Protection Assay oder PCR.

Die Erfindung betrifft außerdem Antisense-Moleküle, die gegen die erfindungsgemäße Nukleinsäuresequenz gerichtet sind und die Expression des MATER-Proteins unterdrücken können. Derartige Moleküle können gezielt zur Kontrazeption eingesetzt werden.

Weiterhin sind Gegenstand der Erfindung Vektoren die mindestens eine Kopie der erfindungsgemäßen Nukleinsäure enthalten. Vektoren können prokaryontische oder eukaryontische Vektoren sein. Beispiele für Vektoren sind pPRO (Clontech), pBAD (Invitrogen), pSG5 (Stratagene), pCl (Promega), pIRES (Clontech), pBAC (Clontech), pMET (Invitrogen), pBlueBac (Inyitrogen). In diese Vektoren können mit den dem Fachmann bekannten Methoden die erfindungsgemäßen Nukleinsäuren eingefügt werden. Die erfindungsgemäßen Nukleinsäuren befinden sich vorzugsweise in Verbindung mit Expressionssignalen wie z.B. Promotor und Enhancer auf dem Vektor.

Die Erfindung betrifft ferner Zellen, die mit einer erfindungsgemäßen Nukleinsäuresequenz oder mit einem erfindungsgemäßen Vektor transfiziert sind. Als Zellen können z.B. *E. coli,* Hefe, *Pichia,* Sf9, COS, CV-1 oder BHK verwendet werden. Diese Zellen können für die Produktion des erfindungsgemäßen Polypeptids oder für Testsysteme verwendet werden.

In den USA leiden 1% der Frauen an ,autoimmune premature ovarian failure', eine Erkrankung deren klinisches Syndrom durch die Ausbildung von Amenorrhoe (<40 Jahre), durch Infertilität und durch Symptome der Menopause, verursacht durch Hypoestrogenämie und Hypergonadotropinämie, charakterisiert ist (Nelson and Tong, Endocrinology, 1999, 140, 3720 - 3726). In einem analogen Maus-Modell der Autoimmun-Oophoritis konnte gezeigt werden, dass eine der Ursachen für diese Erkrankung die Ausbildung von Antikörpern gegen das Maus-Mater-Protein ist. Ein Gegenstand der Erfindung ist deshalb die Verwendung der erfindungsgemäßen Polypeptide oder der dafür kodierenden Nukleinsäuren als Zielsubstanz zur Herstellung eines Mittels zur Behandlung von Fertilitätsstörungen, deren Ursache in einer Fehlfunktion des MATER-Proteins liegt.
Insbesondere erfasst die Erfindung die Verwendung
a. einer erfindunggemäßen Nukleinsäure,
b. eines erfindungsgemäßen Polypeptids, oder
c. einer erfindungsgemäßen Zelle
zur Identifizierung von Effektoren eines erfindungsgemäßen Polypeptids. Effektoren sind Substanzen, die auf das erfindungsgemäße Polypeptid inhibitorisch oder aktivierend wirken, und die in der Lage sind, die MATER-Funktion der erfindungsgemäßen Polypeptide zu beeinflußen.

Für die gezielte Kontrazeption kann es von Vorteil sein, Embryos im Zwei-Zell-Stadium festzuhalten um eine weitere Entwicklung zu verhindern. Durch Gabe von Antikörpern, die gegen das erfindungsgemäße Polypeptid gerichtet sind, kann die endogene Funktion von MATER beeinträchtigt und / oder aufgehoben werden. Die Erfindung betrifft deshalb auch die Verwendung von Antikörpern die gegen ein erfindungsgemäßes Polypeptid gerichtet sind.

Die Erfindung betrifft weiterhin ein Testsystem zur Identifizierung von Effektoren eines erfindungsgemäßen Polypeptids, wobei ein erfindungsgemäßes Polypeptid als ganzes oder Teilsequenzen davon mit einem Modulator inkubiert werden kann und beispielsweise die Interaktion von MATER mit Proteinen oder Teilsequenzen anderer Proteine gemessen werden kann (Protein-Interaktionsassay). Die Protein-Protein Interaktionen sollen in dem Mammalian Two-Hybrid Assay System (Stratagene) gemessen werden, in dem die Interaktion zwischen MATER und anderen Proteinen oder Teilsequenzen davon, über die Aktivierung der Expression eines Reporter Gens bestimmt wird.

Die Erfindung betrifft weiterhin ein Testsystem zur Identifizierung von Effektoren eines erfindungsgemäßen Polypeptids, wobei ein erfindungsgemäßes Polypeptid als ganzes oder Teilsequenzen davon mit einem Modulator inkubiert werden kann und beispielsweise die Menge an hydrolysiertem Nukleotid gemessen werden kann. Als Teilsequenz kann z.B. der Bereich, der die "Leucin-rich-repeats" und die NACHT-Domäne umfaßt, oder kürzere Stücke davon verwendet werden. Die Aktivität der Effektoren kann z.B. gemessen werden, indem markiertes NTP verwendet wird und die Spaltung in NDP und Pi gemessen wird. (NTPase-Assay).

Neben der Hydrolyse von NTP kann auch ein Bindungstest durchgeführt werden, um Substanzen zu identifizieren, die die Bindung von NTP an MATER verhindern.
Dieser Bindungstest kann auch mit einer erfindungsgemäßen Zelle durchgeführt werden, die das erfindungsgemäße Polypeptid enthält. Neben der Bindung der zu testenden Substanzen an MATER kann auch ein intrazellulärer Effekt gemessen werden.

Die Effektoren des erfindungsgemäßen Polypeptids können zur Behandlung von Krankheiten, die auf Fehlfunktionen im MATER-Protein beruhen, eingesetzt werden. Beispiele hierfür sind ovarielle Dysfunktion, ,Autoimmune premature ovarian failure', enzündliche Erkrankungen und Erkrankungen des Immunsystems. Außerdem können diese Effektoren zur Behandlung von weiblicher Infertilität eingesetzt werden.

Die Effektoren des erfindungsgemäßen Polypeptids können auch zur Kontrazeption bei der Frau eingesetzt werden. Blockieren Sie die NTPase-Aktivität von Mater so unterläuft die befruchtete Eizelle Apoptose und eine weitere Entwicklung ist nicht mehr möglich.
Die Erfindung betrifft weiterhin ein Testsystem zur Identifizierung von Effektoren eines erfindungsgemäßen Polypeptids, wobei ein erfindungsgemäßes Polypeptid als ganzes oder Teilsequenzen davon mit einem Modulator inkubiert werden kann und beispielsweise Apoptoseinduktion in Zellen gemessen werden kann. Als Teilsequenz kann z.B. der Bereich, der die "Leucin-rich-repeats" und die NACHT-Domäne umfaßt, oder kürzere Stücke davon verwendet werden. Die Aktivität der Effektoren kann z.B. gemessen werden, indem ihr Einfluss auf die Apoptoseinduktion bei Zellen, die mit Mater oder einem Teilstück von Mater inkubiert wurden (Cell-Death Assay).

Es wurde gefunden, dass das erfindungsgemäße Polypeptid ein Antigen darstellt, das unbedingt erforderlich ist für die Entwicklung des Embryos über das Zwei-Zell-Stadium hinaus. Frauen, die unter Fertilitätsstörungen leiden, könnte direkt das Antigen appliziert werden. Eine andere Möglichkeit wäre der Einsatz des Antigens *in vitro* zu einer Oocytenkultur. Eine weitere Möglichkeit wäre der Zugabe des Antigens in das Befruchtungsmedium oder in das Medium zur Kultur des Embryos. Die so präparierten Oocyten werden dann für die *in vitro* Fertilisation verwendet. Eine Behandlung mit Antikörpern gegen das erfindungsgemäße Polypeptid oder Teilstücken davon könnte von Frauen zur Fertilitätskontrolle genutzt werden.

Weiterhin betrifft die Erfindung ein Verfahren zur Bereitstellung eines pharmazeutischen Mittels, wobei
a. Substanzen mit einem erfindungsgemäßen Testsystem in Kontakt gebracht werden,
b. die Wirkung der Substanzen auf das Testsystem im Vergleich zu Kontrollen gemessen wird,
c. eine Substanz, die in Schritt b. eine Modulation der Aktivität der erfindungsgemäßen Polypeptide zeigt, identifiziert wird
d. und die in Schritt c. identifizierte Substanz mit in der Pharmazie üblichen Formulierungsstoffen gemischt wird.
Unter Aktivität des erfindungsgemäßen Polypeptids ist die NTPase Aktivität des Polypeptids zu verstehen oder dessen Eigenschaft Apoptose zu induzieren. Eine Substanz, die durch ein erfindungsgemäßes Verfahren identifiziert wird, kann gegebenenfalls bezüglich metabolischer Stabilität, Aktivität in einem erfindungsgemäßen Testsystem und/oder Bioverfügbarkeit optimiert werden. Dafür können in der Chemie gängige Methoden verwendet werden.
Die bevorzugten Zubereitungen bestehen in einer Darreichungsform, die zur oralen, enteralen oder parenteralen Applikation geeignet ist. Solche Darreichungsformen sind beispielsweise Tabletten, Filmtabletten, Dragees, Pillen, Kapseln, Pulver oder Depotformen sowie Suppositorien. Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmittel wie Carboxypolymethylen, Carboxymethylcellulose, Celluloseacetatphthalat oder Polyvinylacetat, erhalten werden. Die Tabletten können auch aus mehreren Schichten bestehen.

Ferner betrifft die Erfindung Verfahren zur Bestimmung der Autoimmun-Antikörper gegen das MATER-Protein. Autoimmunität ist ein gut beschriebener Mechanismus für das ,Premature ovarian failure', einer Erkrankung, bei der die Ovarien der Frauen von ihrem eigenen Immunsystem angegriffen werden, eine Entzündung der Ovarien ist meist das Resultat. Zur Diagnose von Fertilitätsstörungen können Körperproben (Gewebe oder Flüßigkeiten) bei der Frau untersucht werden. Der Gehalt an Autoimmun-Antikörpern läßt sich mittels Immuntests wie z.B. einem ELISA (enzyme linked immunosorbent assay) oder in Radioimmunotests bestimmen. So kann die Vorhandensein an Autoimmun-Antikörpern in Gewebe- oder Zellextrakten nachgewiesen werden und erlaubt Rückschlüsse auf den Gesundheitszustand des Ovars.

Es wurde auch gefunden, dass die Eizellqualität abhängig ist von der Mater Expression. Daher kann die Mater Expression in Eizellen auch als diagnostischer Marker zur Bestimmung der Eizellqualität eingesetzt werden.

Weiterhin betrifft die Erfindung Verfahren zur Diagnostik von Erkrankungen, deren Ursachen Mutationen des MATER Proteins beinhalten. Hierzu können DNA-Chips verwendet werden. Die Erfindung betrifft daher weiterhin einen DNA-Chip, auf dem mindestens ein Oligonukleotid immobilisiert ist, das der vollständigen cDNA-Sequenz oder einer Teilsequenz bzw. komplementären Sequenz zu der in Seq ID 1 beschriebenen, entspricht. Die Erfindung betrifft somit ferner die Verwendung eines erfindungsgemäßen DNA-Chips zur Diagnose von Fertilitätsstörungen u. a. im Ovar und Endometrium.
DNA-Chips, auch als DNA-Mikroarrays bekannt, sind miniaturisierte Träger, meist aus Glas oder Silizium, auf deren Oberfläche DNA-Moleküle bekannter Sequenz in einem geordneten Raster in hoher Dichte immobilisiert werden. Die Oberflächen-gebundenen DNA-Moleküle werden mit komplementären, eventuell markierten Nukleinsäuren hybridisiert. Die Markierung kann ein Fluoreszenzfarbstoff sein.
Bei Oligonukleotid-Chips stellen die Oligonukleotide, die auf einem erfindungsgemäßen DNA-Chip gebunden sein können, Teilsequenzen der Genprodukte (mRNA bzw. daraus abgeleitete cDNA) dar. Es können ein oder mehrere Oligonukleotide pro Gen auf dem DNA-Chip gebunden sein. Bevorzugt sind 25 Nukleotid-lange Oligonukleotide. Diese werden bevorzugt aus dem jeweiligen 3'untranslatierten Ende des Gens ausgewählt. Methoden zur Herstellung und Verwendung von DNA-Chips sind z.B. in den US-Patenten Nr. 5,578,832; 5,556,752 und 5,510,270 beschrieben.
Bei cDNA-Chips sind die vollständigen Genprodukte (cDNAs) oder Subfragmente (200-500bp lang) auf dem Chip gebunden. Die Methode wird z. B. in Eckmann L *et al.,* J. Biol. Chem., 2000, 275(19), 14084-14094 beschrieben.

Zuerst werden die geeigneten DNA-Sequenzen gemäß Seq ID NO 1 oder Seq ID NO 3, bestimmt. Geeignet sind Sequenzen, die mit den ausgewählten Gentranskripten hybridisieren können. Die Oligonukleotide werden dann durch einen chemischen Prozess, der auf photolithographischen Verfahren basiert, auf dem Chip hergestellt. Dazu werden photolithographische Masken verwendet, die durch geeignete Computer-Algorithmen erzeugt wurden.
Die markierte RNA wird mit dem Chip in einem Hybridisierungsofen inkubiert. Anschliessend wird der Chip in einem Scanner analysiert, der die Hybridisierungsprofile bestimmt. Dadurch kann festgestellt werden, ob Veränderungen des Transkripts erfolgt sind (z.B. Mutationen, Trunkierungen). Ebenso ermöglicht es die Quantifizierung des Tranaskripts und so des MATER-Proteins und ermöglicht Ruckschlüsse auf z.B. eine Mutation im Promoter.
Es wurde gefunden, dass Mater während des Implantationsfensters (LH +8) vermindert exprimiert wird im Vergleich zur prärezeptiven Phase (LH +4). Durch Bestimmung der MATER-Expression kann demnach der optimale Zeitpunkt für die Implantation von Oocyten, die *in vitro* fertilisiert wurden, bestimmt werden. Zudem kann die Bestimmug der Mater Expression als diagnostischer Marker zur Bestimmung des Implantationsfensters im Endometrium eingesetzt werden.

### Beschreibung der Abbildungen

Fig. 1 zeigt ein multiples Sequenzalignment der MATER-Proteine von Maus und Mensch.

Fig. 2 gibt die Lokalisation der verschiedenen Domänen innerhalb des humanen MATER-Proteins an. Die Spalten "from" und "to" beziehen sich auf die Aminosäurennummerierung gemäß Seq ID2.

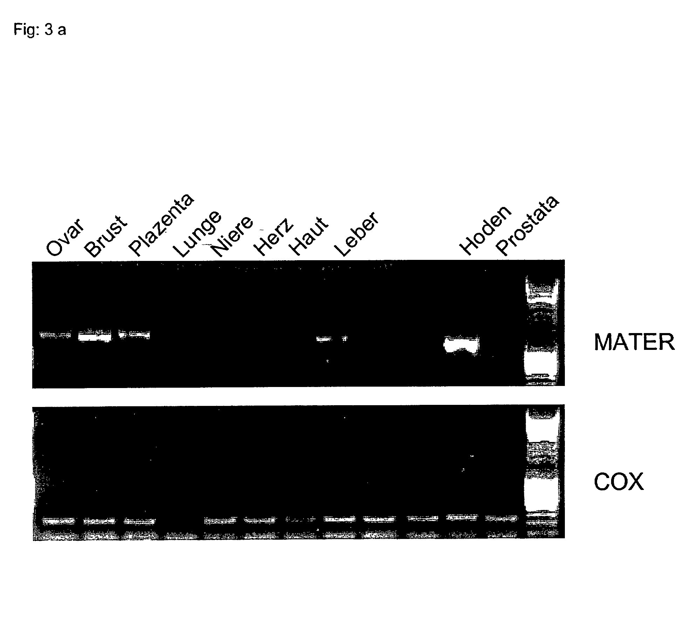

Fig. 3a zeigt das Expressionsmuster der mRNA des humanen MATER-Proteins in verschiedenen Geweben nachgewiesen durch die PCR. Als interne Kontrolle wurden Primer für die Cyclooxygenase (COX) verwendet. Nach der PCR-Amplifizierung wurden die Produkte auf einem Agarose-Gel getrennt und mit Ethidiumbromid gefärbt.

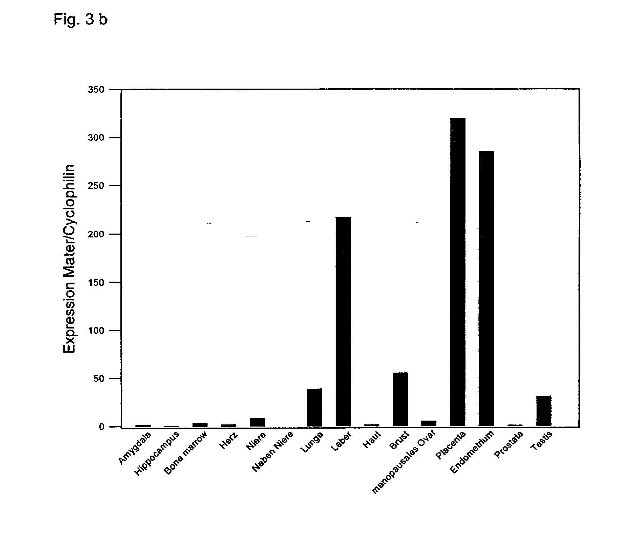

Fig. 3b zeigt die Genexpression der RNA von MATER in verschiedenen Geweben, nachgewiesen durch die real time quantitative PCR -Methode. Es ist sehr deutlich zu erkennen, dass MATER in der Placenta und dem Endometrium stark exprimiert wird. Im Ovar wird MATER nur schwach exprimiert. Es handelt sich hier um Ovarien von menopausalen Patientinnen. In diesen Ovarien sind nur noch sehr wenige Eizellen vorhanden und diese Eizellen sind zusätzlich von minderer Qualität, so dass eine geringe Expression von Mater vorliegt.

Fig. 4 zeigt die Genexpression der RNA von MATER im Endometrium nachgewiesen durch die real time quantitative PCR-Methode. Eine stark erniedrigte Regulation des humanen MATER-Gens in der Gruppe (LH+8) im Vergleich zur Gruppe LH+4 ist eindeutig. Spezifische "Primer" für das MATER wurden benutzt. Gruppe LH +4: vor der Öffnung des Implantationsfensters; Gruppe LH +8: Zeit der geöffneten Implantationsfensters bei gesunden Frauen; Gruppe LH +8 EMT: Zeit der geöffneten Implantationsfensters bei an Endometriose erkrankten Frauen; LH: luteinisierendes Hormon; 4 bzw. 8: Zeitraum in Tagen

### Beispiele

Die in den Beispielen verwendeten molekularbiologischen Methoden wie z.B. Polymerase-Kettenreaktion (PCR), Herstellung von cDNA, Klonierung von DNA, Sequenzierung von DNA, wurden in bekannten Lehrbüchern wie zum Beispiel in Molecular Cloning, A Laboratory Manual (Sambrook, J. *et al*., 1989, Cold Spring Habor Laboratory Press) beschrieben, durchgeführt.

### Beispiel 1: Klonierung, Expression und Reinigung des humanen Mater-Proteins.

Für die Expression der beiden Splice-Varianten des Mater-Proteins wurde der kodierende Bereich mittels Polymerasekettenreaktion (Reaktionsbedingungen siehe Bsp2) amplifiziert (Primer für den N-terminalen Bereich: 5' ATGGAAGGAGACAAATCGCTC 3'; Primer für den C-terminalen Bereich 5' TAGTTGGCATTCTTTTGATG 3'), in den Baculovirusexpressionsvektor pBlueBac4.5/V5-His-TOPO (Invitrogen) bzw. den eukaryontischen Expressionsvektor pcDNA3.1/V5/His-TOPO (Invitrogen) eingefügt. Um Nachweis und Reinigung zu vereinfachen, erfolgte eine Fusion mit einem His-Tag. Nach Cotransfektion von Insektenzellen mit der Bac-N-Blue DNA wurden rekombinante Viren erzeugt, die durch eine PCR-Verfahren identifiziert wurden. Ein Phagenstock wurde dann angelegt und für weitere Transfektionen und Produktion des Mater in größeren Mengen verwendet. Die Reinigung der His-getaggten Proteine erfolgte über eine Nickel- Affinitätssäule.

### Beispiel 2: Untersuchungen zur Expression der mRNA des humanen Mater-Proteins.

a) Zur Untersuchung der Expression der mRNA wurde eine PCR wie folgt durchgeführt. Als Template wurde c-DNA von folgenden Geweben verwendet: Knochen, Samenleiter, Prostata, Hoden, Placenta, Brust, Ovar, Nebenniere, Haut, Niere und Lunge. Der Reaktionsansatz enthält: 2 µl cDNA; 20 µM Primer (antisense Primer: 5' cacatgaacatccttctccc 3'; sense primer: 5'cacagtcctccagtatcagc 3'), 10 mM d'NTP; 1,5 mM MgCl₂ und 0,5U Taq Gold (Perkin Elmer). Die PCR- Konditionen sind 94°C 10 min dann 40 Zyklen 94°C 1 min; 58°C 1 min; 72°C 1,5 min. Anschließend wurde ein Aliquot auf ein 1% Agarosegel in 1X TAE-Laufpuffer aufgetragen. Unter diesen Bedingungen konnte eine Expression der mRNA in folgenden Geweben gefunden werden: Testis, Placenta und Ovar (siehe Figur 3a).
b) Bestimmung der MATER-RNA Mengen in endometrialen Proben durch Real-time quantitative RT-PCR Analyse:
Endometrium wurde von Patientinnen, die den drei Gruppen LH+4, LH+8, LH+8/Endometriose gehören, entnommen und in flüssigem Stickstoff schockgefroren. Aus den durch "Mörsern" unter flüssigem Stickstoff pulverisierten Geweben wurde mittels TRIZOL (Invitrogen) die Gesamt-RNA isoliert. Ausgehend von 5 µg Gesamt-RNA wurde zunächst ein DNase I-Verdau (Invitrogen) und anschließend eine Erststrang-Synthese unter Verwendung des SUPER-SCRIPT First-Strand Synthesis System für RT-PCR (Invitrogen) durchgeführt. Für die Amplifizierung der Transkripte zur relativen Quantifizierung wurden 0,125 µl Erststrang-DNA eingesetzt. Unter Verwendung von human Mater spezifischen Primer Paaren (forward Primer: 5'CCT CCC AAG TTG AGG GAT CTT-3' und reversere Primer: 5'TAC CCC TGG TGT GCA GCA C-3') wurde die Amplifizierung unter folgenden PCR-Bedingungen durchgeführt: 10 Min. 95 °C; 15 Sek. 95 °C, 1 Min. 60 °C (40 Zyklen). Als interne Kontrolle wurden Primer für human Cyclophilin (huCYC) Part Number 4310857 (PE Biosystems) in der PCR eingesetzt. Die Messung der Fluorescens als Maß für die Zunahme von Amplifizierungsprodukte erfolgte online mittels ABI Prism 7700 Sequence Detector (PE Biosystems). Die Reinheit der Amplifizierungsprodukte wurde durch die Aufnahme von Schmelzkurven überprüft. (siehe Figur 3b und 4)

### Beispiel 3: Testsystem zum Auffinden von Substanzen, die die NTPase Aktivität beeinflussen.

Ein Standard NTPase Assay wurde folgendermaßen durchgeführt: Inkubation für 30 min bei 30°C. 5-30 pMol des gereinigten Mater-Proteins oder eines Fragmentes, das die NTPase Domäne umschließt, wurden in einem Reaktionspuffer [20 mM Tris/HCL pH7,5; 3 mM MgCl₂; 1 mM 2-mercaptoethanol; 10%Glycerol; 0,01% Triton X-100; 0,1mg/ml BSA; 11 µM[γ-³² P]NTP (0,5µCi)] inkubiert (Endvolumen 25 µl). Die Reaktion wurde durch Zugabe von 0,5 mL aktivierter Kohle (2mg/ml) gestoppt. Anschließend wurde der Ansatz 10 min bei 10000 x g zentrifugiert und 50 µl des Überstandes im Szintillationszähler (Cerenkov) gezählt. Substanzen, die die NTPase-Aktivität von Mater beeinflussen, werden dem Reaktionspuffer zugegeben.

### Beispiel 4: Testsystem zum Auffinden von Substanzen, die die Nukleotidbindung an Mater verhindern.

Der Reaktionsmix [20 mM Tris/Hcl pH7.5; 3 mM MgCl₂, 1 mM 2-Mercaptoethanol; 10% Glycerol, 0,01% Triton X-100, 0,1mg/ml BSA und 50µM [γ-³²-P]ATP (0,5µCi)] mit 30-80 pMol gereinigtem Mater-Protein oder einem Fragment des Mater-Proteins, das die ATP-Bindungsstelle umfasst, wurde 30 min bei 30°C inkubiert. Die Reaktion wurde durch Zugabe von eiskaltem NaCl/Pᵢ beendet und die Proben wurden durch BA85 Nitrocellulose-Filter microfiltriert. Die Nirotrozellulose wurde anschließend 2mal mit 2 mL NaCl/Pᵢ gewaschen, getrocknet und das gebundene [γ-³²-P]ATP nach Cerenkov gezählt. Substanzen, die die Bindung beeinflussen, werden dem Reaktionsmix zugefügt.

### Beispiel 5: Testsystem zum Auffinden von Substanzen, die Apoptose induzieren (Cell death Assay)

Zum Auffinden von Substanzen, die Apoptose induzieren, wurde das in einen eukaryontischen Expressionsvektor (pCDNA3.1 siehe Bsp1) klonierte Mater oder Teilsequenzen von Mater in eine eukaryontischen Zelle, z.B. MCF-7 mittels LipfectAMINE (Life Technology Inc) nach den Angaben des Herstellters transfiziert. 24h nach der Transfektion wurden die Zellen in 0,5% Glutaraldehyd fixiert und mit 5-bromo-4-chloro-3-indolyl β-D galactopyranoside (X-gal) für 4h inkubiert. Die Zellen wurden im Phasenkontrastmikroskop visualisiert und der Anteil apoptotischer Zellen wurde ausgezählt. Substanzen, die die Apoptoseinduktion beeinflussen werden dem Zellkulturmedium (10%hitzeinaktviertes Fötales Kälberserum in RPMI1640) zugegeben.

## Patentansprüche

1. Nukleinsäure umfassend
a. die in Seq ID NO 1 oder Seq ID NO 3 dargestellte Nukleotidsequenz,
b. eine einer Sequenz aus a. im Rahmen der Degeneration des genetischen Codes entsprechende Nukleotidsequenz oder
c. eine mit den Sequenzen aus a. oder b. unter stringenten Bedingungen hybridisierende Nukleotidsequenz mit der Funktion eines humanen MATER-Proteins.

2. Nukleinsäure nach Anspruch 1 umfassend einen Protein-kodierenden Abschnitt der der in Seq ID NO 1 oder Seq ID NO 3 dargestellten Nukleinsäuresequenz.

3. Nukleinsäure, kodierend für ein Polypeptid mit der in Seq ID NO 2 oder Seq ID NO 4 dargestellten Aminosäuresequenz.

4. Polypeptide kodiert von einer Nukleinsäure nach einem der Ansprüche 1-3.

5. Polypeptid, umfassend die in Seq ID NO 2 oder in Seq ID NO 4 dargestellte Aminosäuresequenz oder Teile davon.

6. Verwendung eines Polypeptids nach Anspruch 4 und 5 oder von Teilen dieses Polypeptids zur Herstellung von Antikörpern.

7. Antikörper gegen ein Polypeptid nach einem der Ansprüche 4 und 5.

8. Verwendung einer Sonde mit Nukleinsäuresequenzen, die komplementär zu den Nukleinsäuresequenzen nach Anspruch 1-3 sind, zur Herstellung einer Reagenz zum Nachweis der Gegenwart von mRNA und nach einem der Ansprüche 1-3 in Zellen.

9. Antisense-Molekül, das gegen die Nukleinsäure gemäß Anspruch 1 gerichtet ist.

10. Vektor enthaltend mindestens eine Kopie einer Nukleinsäure nach einem der Ansprüche 1-3.

11. Zelle transfiziert mit einer Nukleinsäure nach einem der Ansprüche 1-3 oder mit einem Vektor nach Anspruch 9.

12. Verwendung einer Zelle nach Anspruch 11 zur Expression der Nukleinsäure nach einem der Ansprüche 1-3.

13. Verwendung
a. einer Nukleinsäure nach einem der Ansprüche 1 bis 3,
b. eines Polypeptids nach einem der Ansprüche 4 und 5 oder
c. einer Zelle nach Anspruch 11
zur Identifizierung von Effektoren eines Polypeptids nach Anspruch 4 oder 5.

14. Verwendung einer Nukleinsäure nach Anspruch 1 bis 3 oder eines Polypeptids nach Anspruch 4 und 5 oder des Antikörpers gemäß Anspruch 7 oder eines Antisense-Moleküls nach Anspruch 9 als Zielsubstanz zur Herstellung eines Mittels für Krankheiten, die ursächlich mit dem MATER-Gen und / oder Protein zusammenhängen.

15. Testsystem zur Identifizierung von Effektoren eines Polypeptids nach Anspruch 4 oder 5, wobei ein erfindungsgemäßes Polypeptid als ganzes oder Teilsequenzen davon mit Domänen anderer Proteine in Kontakt gebracht wird und deren Interaktion gemessen wird.

16. Testsystem nach Anspruch 15, wobei die Effektoren die Interaktion der Polypeptide oder Teilsequenzen mit Domänen anderer Proteine aktivieren oder inhibieren.

17. Testsystem zur Identifizierung von Effektoren eines Polypeptids nach Anspruch 4 oder 5, wobei ein erfindungsgemäßes Polypeptid als ganzes oder Teilsequenzen davon mit einem Modulator (Effektor) inkubiert werden und die Menge an hydrolysiertem NTP detektiert wird.

18. Testsystem nach Anspruch 14 oder 17 wobei die Effektoren die NTPase Aktivität inhibieren oder aktivieren.

19. Testsystem zur Identifizierung von Effektoren der NTPase Aktivität des erfindunggemäßen Polypeptids nach Anspruch 17 in einer erfindungsgemäßen Zelle mit Messung der intrazellulären Phosphoylierung.

20. Verfahren zur Bereitstellung eines pharmazeutischen Mittels, wobei
a. Substanzen mit einem Testsystem nach Anspruch 17 - 19 in Kontakt gebracht werden.
b. die Wirkung der Substanzen auf das Testsystem im Vergleich zu Kontrollen gemessen wird
c. eine Substanz, die in Schritt b. eine Modulation der Aktivität des Polypeptids gemäß Anspruch 4 oder 5 zeigt, identifiziert wird
d. und die in Schritt c. identifizierte Substanz mit in der Pharmazie üblichen Formulierungsstoffen gemischt wird.

21. Diagnose-Verfahren zur Bestimmung von Autoimmun-Antikörpern gegen MATER in Körperproben (Gewebe, Flüssigkeiten).

22. Diagnose-Verfahren zur Bestimmung der Menge an Mater-Protein oder Mater mRNA in Eizellen.

23. DNA-Chip, **dadurch gekennzeichnet, dass** mindestens ein Oligonukleotid immobilisiert ist, das der vollständigen cDNA-Sequenz oder einer Teilsequenz bzw. komplementären Sequenz zu der in Seq ID NO 1 oder Seq ID NO 3 beschriebenen, entspricht.

24. Verwendung eines DNA-Chips nach Anspruch 20 zur Diagnose von Fertilitätsstörungen.

25. Verwendung von Modulatoren der Mater Expression zur Behandlung von durch Endometriose bedingten Fertilitätsstörungen.

26. Methode zur Inhibierung der Aktivität des erfindungsgemäßen Polypeptids nach Anspruch 4 oder 5 in einer Zelle umfassend das Inkontaktbringen des erfindungsgemäßen Polypeptids mit einem Effektor auffindbar mit einem Testsystem nach Anspruch 17 - 19.


SEQUENZPROTOKOLL

<110> SCHERING AKTIENGESELLSCHAFT

<120> Humanes Mater Protein

<130> 52174A

<140>
<141>

<160> 4

<170> PatentIn Ver. 2.1

<210> 1
<211> 3926
<212> DNA
<213> Homo sapiens

<400> 1
   atggaaggag acaaatcgct caccttttcc agctacgggc tgcaatggtg tctctatgag 60
   ctagacaagg aagaatttca gacattcaag gaattactaa agaagaaatc ttcagaatcg 120
   accacatgct ctattccaca gtttgaaatc gagaatgcca acgtggaatg tctggcactc 180
   ctcttgcatg agtattatgg agcatcgctg gcctgggcta cgtccattag catctttgaa 240
   aacatgaacc tgcgaaccct ctcggagaag gcacgggatg acatgaaaaa ttcaccagaa 300
   gatcctgaag caacgatgac tgaccaagga ccaagcaagg aaaaagtgcc agaaaataaa 360
   tatggcatga ctaagcttat cttgggggtg tctgacatct ctgactcgaa taataaacac 420
   aagtatgttg gaattcattc ttcttttgca gaaatttcac aagctatgga acaagaaggt 480
   gccacagcag cagagacaga agaacaagaa atttcacaag ctatggaaca agaaggtgcc 540
   acagcagcag agacagaaga acaaggacat ggaggtgaca catgggacta caagagtcac 600
   gtgatgacca aattcgctga ggaggaggat gtacgtcgta gttttgaaaa cactgctgct 660
   gactggccgg aaatgcaaac gttggctggt gcttttgatt cagaccggtg gggcttccgg 720
   cctcgcacgg tggttctgca cggaaagtca ggaattggga aatcggctct agccagaagg 780
   atcgtgctgt gctgggcgca aggtggactc taccagggaa tgttctccta cgtcttcttc 840
   ctccccgtta gagagatgca gcggaagaag gagagcagtg tcacagagtt catctccagg 900
   gagtggccag actcccaggc tccggtgacg gagatcatgt cccgaccaga aaggctgttg 960
   ttcatcattg acggtttcga tgacctgggc tctgtcctca acaatgacac aaagctctgc 1020
   aaagactggg ctgagaagca gcctccgttc accctcatac gcagtctgct gaggaaggtc 1080
   ctgctccctg agtccttcct gatcgtcacc gtcagagacg tgggcacaga gaagctcaag 1140
   tcagaggtcg tgtctccccg ttacctgtta gttagaggaa tctccgggga acaaagaatc 1200
   cacttgctcc ttgagcgcgg gattggtgag catcagaaga cacaagggtt gcgtgcgatc 1260
   atgaacaacc gtgagctgct cgaccagtgc caggtgcccg ccgtgggctc tctcatctgc 1320
   gtggccctgc agctgcagga cgtggtgggg gagagcgtcg cccccttcaa ccaaacgctc 1380
   acaggcctgc acgccgcttt tgtgtttcat cagctcaccc ctcgaggcgt ggtccggcgc 1440
   tgtctcaatc tggaggaaag agttgtcctg aagcgcttct gccgtatggc tgtggaggga 1500
   gtgtggaata ggaagtcagt gtttgacggt gacgacctca tggttcaagg actcggggag 1560
   tctgagctcc gtgctctgtt tcacatgaac atccttctcc cagacagcca ctgtgaggag 1620
   tactacacct tcttccacct cagtctccag gacttctgtg ccgccttgta ctacgtgtta 1680
   gagggcctgg aaatcgagcc agctctctgc cctctgtacg ttgagaagac aaagaggtcc 1740
   atggagctta aacaggcagg cttccatatc cactcgcttt ggatgaagcg tttcttgttt 1800
   ggcctcgtga gcgaagacgt aaggaggcca ctggaggtcc tgctgggctg tcccgttccc 1860
   ctgggggtga agcagaagct tctgcactgg gtctctctgt tgggtcagca gcctaatgcc 1920
   accaccccag gagacaccct ggacgccttc cactgtcttt tcgagactca agacaaagag 1980
   tttgttcgct tggcattaaa cagcttccaa gaagtgtggc ttccgattaa ccagaacctg 2040
   gacttgatag catcttcctt ctgcctccag cactgtccgt atttgcggaa aattcgggtg 2100
   gatgtcaaag ggatcttccc aagagatgag tccgctgagg catgtcctgt ggtccctcta 2160
   tggatgcggg ataagaccct cattgaggag cagtgggaag atttctgctc catgcttggc 2220
   acccacccac acctgcggca gctggacctg ggcagcagca tcctgacaga gcgggccatg 2280
   aagaccctgt gtgccaagct gaggcatccc acctgcaaga tacagaccct gatgtttaga 2340
   aatgcacaga ttacccctgg tgtgcagcac ctctggagaa tcgtcatggc caaccgtaac 2400
   ctaagatccc tcaacttggg aggcacccac ctgaaggaag aggatgtaag gatggcgtgt 2460
   gaagccttaa aacacccaaa atgtttgttg gagtctttga ggctggattg ctgtggattg 2520
   acccatgcct gttacctgaa gatctcccaa atccttacga cctcccccag cctgaaatct 2580
   ctgagcctgg caggaaacaa ggtgacagac cagggagtaa tgcctctcag tgatgccttg 2640
   agagtctccc agtgcgccct gcagaagctg atactggagg actgtggcat cacagccacg 2700
   ggttgccaga gtctggcctc agccctcgtc agcaaccgga gcttgacaca cctgtgccta 2760
   tccaacaaca gcctggggaa cgaaggtgta aatctactgt gtcgatccat gaggcttccc 2820
   cactgtagtc tgcagaggct gatgctgaat cagtgccacc tggacacggc tggctgtggt 2880
   tttcttgcac ttgcgcttat gggtaactca tggctgacgc acctgagcct tagcatgaac 2940
   cctgtggaag acaatggcgt gaagcttctg tgcgaggtca tgagagaacc atcttgtcat 3000
   ctccaggacc tggagttggt aaagtgtcat ctcaccgccg cgtgctgtga gagtctgtcc 3060
   tgtgtgatct cgaggagcag acacctgaag agcctggatc tcacggacaa tgccctgggt 3120
   gacggtgggg ttgctgcact gtgcgaggga ctgaagcaaa agaacagtgt tctgacgaga 3180
   ctcgggttga aggcatgtgg actgacttct gattgctgtg aggcactctc cttggccctt 3240
   tcctgcaacc ggcatctgac cagtctaaac ctggtgcaga ataacttcag tcccaaagga 3300
   atgatgaagc tgtgttcggc ctttgcctgt cccacgtcta acttacagat aattgggctg 3360
   tggaaatggc agtaccctgt gcaaataagg aagctgctgg aggaagtgca gctactcaag 3420
   ccccgagtcg taattgacgg tagttggcat tcttttgatg aagatgaccg gtactggtgg 3480
   aaaaactgaa gatacggaaa cctgccccac tcacacccat ctgatggagg aactttaaac 3540
   gctgttttct cagagcaagc tatgcacctg ggagttcctt ctcaaagatg gagaatgatt 3600
   tctgattctc acaaagccct caatggtagt gattcttctg tgttcactct acgttggtta 3660
   ctggatttga aggctagaga ccttcaagtc ataggactca gtatctgtga aatgtccgtc 3720
   atatctcaga gcatatagag ggaattaaat aaacacaaag catttggaaa agttgtcaag 3780
   tggttttctt aactagtgga gatatggttt aggagcagag aggttgggag gacctagatc 3840
   ttcaaaagaa gcccctgaat ttgggtacca caactagtgg tggttttttg ttttttgtgt 3900
   ttttcttttt gttttttggt tttttt 3926

<210> 2
<211> 1162
<212> PRT
<213> Homo sapiens

<400> 2
Met Glu Gly Asp Lys Ser Leu Thr Phe Ser Ser Tyr Gly Leu Gln Trp
1               5                   10                  15

Cys Leu Tyr Glu Leu Asp Lys Glu Glu Phe Gln Thr Phe Lys Glu Leu
            20                  25                  30

Leu Lys Lys Lys Ser Ser Glu Ser Thr Thr Cys Ser Ile Pro Gln Phe
        35                  40                  45

Glu Ile Glu Asn Ala Asn Val Glu Cys Leu Ala Leu Leu Leu His Glu
    50                  55                  60

Tyr Tyr Gly Ala Ser Leu Ala Trp Ala Thr Ser Ile Ser Ile Phe Glu
65                  70                  75                  80

Asn Met Asn Leu Arg Thr Leu Ser Glu Lys Ala Arg Asp Asp Met Lys
                85                  90                  95

Asn Ser Pro Glu Asp Pro Glu Ala Thr Met Thr Asp Gln Gly Pro Ser
            100                 105                 110

Lys Glu Lys Val Pro Glu Asn Lys Tyr Gly Met Thr Lys Leu Ile Leu
        115                 120                 125

Gly Val Ser Asp Ile Ser Asp Ser Asn Asn Lys His Lys Tyr Val Gly
    130                 135                 140

Ile His Ser Ser Phe Ala Glu Ile Ser Gln Ala Met Glu Gln Glu Gly
145                 150                 155                 160

Ala Thr Ala Ala Glu Thr Glu Glu Gln Glu Ile Ser Gln Ala Met Glu
                165                 170                 175

Gln Glu Gly Ala Thr Ala Ala Glu Thr Glu Glu Gln Gly His Gly Gly
            180                 185                 190

Asp Thr Trp Asp Tyr Lys Ser His Val Met Thr Lys Phe Ala Glu Glu
        195                 200                 205

Glu Asp Val Arg Arg Ser Phe Glu Asn Thr Ala Ala Asp Trp Pro Glu
    210                 215                 220

Met Gln Thr Leu Ala Gly Ala Phe Asp Ser Asp Arg Trp Gly Phe Arg
225                 230                 235                 240

Pro Arg Thr Val Val Leu His Gly Lys Ser Gly Ile Gly Lys Ser Ala
                245                 250                 255

Leu Ala Arg Arg Ile Val Leu Cys Trp Ala Gln Gly Gly Leu Tyr Gln
            260                 265                 270

Gly Met Phe Ser Tyr Val Phe Phe Leu Pro Val Arg Glu Met Gln Arg
        275                 280                 285

Lys Lys Glu Ser Ser Val Thr Glu Phe Ile Ser Arg Glu Trp Pro Asp
    290                 295                 300

Ser Gln Ala Pro Val Thr Glu Ile Met Ser Arg Pro Glu Arg Leu Leu
305                 310                 315                 320

Phe Ile Ile Asp Gly Phe Asp Asp Leu Gly Ser Val Leu Asn Asn Asp
                325                 330                 335

Thr Lys Leu Cys Lys Asp Trp Ala Glu Lys Gln Pro Pro Phe Thr Leu
            340                 345                 350

Ile Arg Ser Leu Leu Arg Lys Val Leu Leu Pro Glu Ser Phe Leu Ile
        355                 360                 365

Val Thr Val Arg Asp Val Gly Thr Glu Lys Leu Lys Ser Glu Val Val
    370                 375                 380

Ser Pro Arg Tyr Leu Leu Val Arg Gly Ile Ser Gly Glu Gln Arg Ile
385                 390                 395                 400

His Leu Leu Leu Glu Arg Gly Ile Gly Glu His Gln Lys Thr Gln Gly
                405                 410                 415

Leu Arg Ala Ile Met Asn Asn Arg Glu Leu Leu Asp Gln Cys Gln Val
            420                 425                 430

Pro Ala Val Gly Ser Leu Ile Cys Val Ala Leu Gln Leu Gln Asp Val
        435                 440                 445

Val Gly Glu Ser Val Ala Pro Phe Asn Gln Thr Leu Thr Gly Leu His
    450                 455                 460

Ala Ala Phe Val Phe His Gln Leu Thr Pro Arg Gly Val Val Arg Arg
465                 470                 475                 480

Cys Leu Asn Leu Glu Glu Arg Val Val Leu Lys Arg Phe Cys Arg Met
                485                 490                 495

Ala Val Glu Gly Val Trp Asn Arg Lys Ser Val Phe Asp Gly Asp Asp
            500                 505                 510

Leu Met Val Gln Gly Leu Gly Glu Ser Glu Leu Arg Ala Leu Phe His
        515                 520                 525

Met Asn Ile Leu Leu Pro Asp Ser His Cys Glu Glu Tyr Tyr Thr Phe
    530                 535                 540

Phe His Leu Ser Leu Gln Asp Phe Cys Ala Ala Leu Tyr Tyr Val Leu
545                 550                 555                 560

Glu Gly Leu Glu Ile Glu Pro Ala Leu Cys Pro Leu Tyr Val Glu Lys

                565                 570                 575

Thr Lys Arg Ser Met Glu Leu Lys Gln Ala Gly Phe His Ile His Ser
            580                 585                 590

Leu Trp Met Lys Arg Phe Leu Phe Gly Leu Val Ser Glu Asp Val Arg
        595                 600                 605

Arg Pro Leu Glu Val Leu Leu Gly Cys Pro Val Pro Leu Gly Val Lys
    610                 615                 620

Gln Lys Leu Leu His Trp Val Ser Leu Leu Gly Gln Gln Pro Asn Ala
625                 630                 635                 640

Thr Thr Pro Gly Asp Thr Leu Asp Ala Phe His Cys Leu Phe Glu Thr
                645                 650                 655

Gln Asp Lys Glu Phe Val Arg Leu Ala Leu Asn Ser Phe Gln Glu Val
            660                 665                 670

Trp Leu Pro Ile Asn Gln Asn Leu Asp Leu Ile Ala Ser Ser Phe Cys
        675                 680                 685

Leu Gln His Cys Pro Tyr Leu Arg Lys Ile Arg Val Asp Val Lys Gly
    690                 695                 700

Ile Phe Pro Arg Asp Glu Ser Ala Glu Ala Cys Pro Val Val Pro Leu
705                 710                 715                 720

Trp Met Arg Asp Lys Thr Leu Ile Glu Glu Gln Trp Glu Asp Phe Cys
                725                 730                 735

Ser Met Leu Gly Thr His Pro His Leu Arg Gln Leu Asp Leu Gly Ser
            740                 745                 750

Ser Ile Leu Thr Glu Arg Ala Met Lys Thr Leu Cys Ala Lys Leu Arg
        755                 760                 765

His Pro Thr Cys Lys Ile Gln Thr Leu Met Phe Arg Asn Ala Gln Ile
    770                 775                 780

Thr Pro Gly Val Gln His Leu Trp Arg Ile Val Met Ala Asn Arg Asn
785                 790                 795                 800

Leu Arg Ser Leu Asn Leu Gly Gly Thr His Leu Lys Glu Glu Asp Val
                805                 810                 815

Arg Met Ala Cys Glu Ala Leu Lys His Pro Lys Cys Leu Leu Glu Ser
            820                 825                 830

Leu Arg Leu Asp Cys Cys Gly Leu Thr His Ala Cys Tyr Leu Lys Ile
        835                 840                 845

Ser Gln Ile Leu Thr Thr Ser Pro Ser Leu Lys Ser Leu Ser Leu Ala
    850                 855                 860

Gly Asn Lys Val Thr Asp Gln Gly Val Met Pro Leu Ser Asp Ala Leu
865                 870                 875                 880

Arg Val Ser Gln Cys Ala Leu Gln Lys Leu Ile Leu Glu Asp Cys Gly
                885                 890                 895

Ile Thr Ala Thr Gly Cys Gln Ser Leu Ala Ser Ala Leu Val Ser Asn
            900                 905                 910

Arg Ser Leu Thr His Leu Cys Leu Ser Asn Asn Ser Leu Gly Asn Glu
        915                 920                 925

Gly Val Asn Leu Leu Cys Arg Ser Met Arg Leu Pro His Cys Ser Leu
    930                 935                 940

Gln Arg Leu Met Leu Asn Gln Cys His Leu Asp Thr Ala Gly Cys Gly
945                 950                 955                 960

Phe Leu Ala Leu Ala Leu Met Gly Asn Ser Trp Leu Thr His Leu Ser
                965                 970                 975

Leu Ser Met Asn Pro Val Glu Asp Asn Gly Val Lys Leu Leu Cys Glu
            980                 985                 990

Val Met Arg Glu Pro Ser Cys His Leu Gln Asp Leu Glu Leu Val Lys
        995                 1000                1005

Cys His Leu Thr Ala Ala Cys Cys Glu Ser Leu Ser Cys Val Ile Ser
    1010                1015                1020

Arg Ser Arg His Leu Lys Ser Leu Asp Leu Thr Asp Asn Ala Leu Gly
1025                1030                1035                1040

Asp Gly Gly Val Ala Ala Leu Cys Glu Gly Leu Lys Gln Lys Asn Ser
                1045                1050                1055

Val Leu Thr Arg Leu Gly Leu Lys Ala Cys Gly Leu Thr Ser Asp Cys
            1060                1065                1070

Cys Glu Ala Leu Ser Leu Ala Leu Ser Cys Asn Arg His Leu Thr Ser
        1075                1080                1085

Leu Asn Leu Val Gln Asn Asn Phe Ser Pro Lys Gly Met Met Lys Leu
    1090                1095                1100

Cys Ser Ala Phe Ala Cys Pro Thr Ser Asn Leu Gln Ile Ile Gly Leu
1105                1110                1115                1120

Trp Lys Trp Gln Tyr Pro Val Gln Ile Arg Lys Leu Leu Glu Glu Val
                1125                1130                1135

Gln Leu Leu Lys Pro Arg Val Val Ile Asp Gly Ser Trp His Ser Phe
            1140                1145                1150

Asp Glu Asp Asp Arg Tyr Trp Trp Lys Asn
        1155                1160

<210> 3
<211> 3830
<212> DNA
<213> Homo sapiens

<400> 3

atggaaggag acaaatcgct caccttttcc agctacgggc tgcaatggtg tctctatgag 60
ctagacaagg aagaatttca gacattcaag gaattactaa agaagaaatc ttcagaatcg 120
accacatgct ctattccaca gtttgaaatc gagaatgcca acgtggaatg tctggcactc 180
ctcttgcatg agtattatgg agcatcgctg gcctgggcta cgtccattag catctttgaa 240
aacatgaacc tgcgaaccct ctcggagaag gcacgggatg acatgaaaaa ttcaccagaa 300
gatcctgaag caacgatgac tgaccaagga ccaagcaagg aaaaagtgcc agaaaataaa 360
tatggcatga ctaagcttat cttgggggtg tctgacatct ctgactcgaa taataaacac 420
aagtatgttg gaattcattc ttcttttgca gaaatttcac aagctatgga acaagaaggt 480
gccacagcag cagagacaga agaacaagga catggaggtg acacatggga ctacaagagt 540
cacgtgatga ccaaattcgc tgaggaggag gatgtacgtc gtagttttga aaacactgct 600
gctgactggc cggaaatgca aacgttggct ggtgcttttg attcagaccg gtggggcttc 660
cggcctcgca cggtggttct gcacggaaag tcaggaattg ggaaatcggc tctagccaga 720
aggatcgtgc tgtgctgggc gcaaggtgga ctctaccagg gaatgttctc ctacgtcttc 780
ttcctccccg ttagagagat gcagcggaag aaggagagca gtgtcacaga gttcatctcc 840
agggagtggc cagactccca ggctccggtg acggagatca tgtcccgacc agaaaggctg 900
ttgttcatca ttgacggttt cgatgacctg ggctctgtcc tcaacaatga cacaaagctc 960
tgcaaagact gggctgagaa gcagcctccg ttcaccctca tacgcagtct gctgaggaag 1020
gtcctgctcc ctgagtcctt cctgatcgtc accgtcagag acgtgggcac agagaagctc 1080
aagtcagagg tcgtgtctcc ccgttacctg ttagttagag gaatctccgg ggaacaaaga 1140
atccacttgc tccttgagcg cgggattggt gagcatcaga agacacaagg gttgcgtgcg 1200
atcatgaaca accgtgagct gctcgaccag tgccaggtgc ccgccgtggg ctctctcatc 1260
tgcgtggccc tgcagctgca ggacgtggtg gggagagcgg tcgccccctt caaccaaacg 1320
ctcacaggcc tgcacgccgc ttttgtgttt catcagctca cccctcgagg cgtggtccgg 1380
cgctgtctca atctggagga aagagttgtc ctgaagcgct tctgccgtat ggctgtggag 1440
ggagtgtgga ataggaagtc agtgtttgac ggtgacgacc tcatggttca aggactcggg 1500
gagtctgagc tccgtgctct gtttcacatg aacatccttc tcccagacag ccactgtgag 1560
gagtactaca ccttcttcca cctcagtctc caggacttct gtgccgcctt gtactacgtg 1620
ttagagggcc tggaaatcga gccagctctc tgccctctgt acgttgagaa gacaaagagg 1680
tccatggagc ttaaacaggc aggcttccat atccactcgc tttggatgaa gcgtttcttg 1740
tttggcctcg tgagcgaaga cgtaaggagg ccactggagg tcctgctggg ctgtcccgtt 1800
cccctggggg tgaagcagaa gcttctgcac tgggtctctc tgttgggtca gcagcctaat 1860
gccaccaccc caggagacac cctggacgcc ttccactgtc ttttcgagac tcaagacaaa 1920
gagtttgttc gcttggcatt aaacagcttc caagaagtgt ggcttccgat taaccagaac 1980
ctggacttga tagcatcttc cttctgcctc cagcactgtc cgtatttgcg gaaaattcgg 2040
gtggatgtca aagggatctt cccaagagat gagtccgctg aggcatgtcc tgtggtccct 2100
ctatggatgc gggataagac cctcattgag gagcagtggg aagatttctg ctccatgctt 2160
ggcacccacc cacacctgcg gcagctggac ctgggcagca gcatcctgac agagcgggcc 2220
atgaagaccc tgtgtgccaa gctgaggcat cccacctgca agatacagac cctgatgttt 2280
agaaatgcac agattacccc tggtgtgcag cacctctgga gaatcgtcat ggccaaccgt 2340
aacctaagat ccctcaactt gggaggcacc cacctgaagg aagaggatgt aaggatggcg 2400

tgtgaagcct taaaacaccc aaaatgtttg ttggagtctt tgaggctgga ttgctgtgga 2460
ttgacccatg cctgttacct gaagatctcc caaatcctta cgacctcccc cagcctgaaa 2520
tctctgagcc tggcaggaaa caaggtgaca gaccagggag taatgcctct cagtgatgcc 2580
ttgagagtct cccagtgcgc cctgcagaag ctgatactgg aggactgtgg catcacagcc 2640
acgggttgcc agagtctggc ctcagccctc gtcagcaacc ggagcttgac acacctgtgc 2700
ctatccaaca acagcctggg gaacgaaggt gtaaatctac tgtgtcgatc catgaggctt 2760
ccccactgta gtctgcagag gctgatgctg aatcagtgcc acctggacac ggctggctgt 2820
ggtttcttgg cacttgcgct tatgggtaac tcatggctga cgcacctgag ccttagcatg 2880
aaccctgtgg aagacaatgg cgtgaagctt ctgtgcgagg tcatgagaga accatcttgt 2940
catctccagg acctggagtt ggtaaagtgt catctcaccg ccgcgtgctg tgagagtctg 3000
tcctgtgtga tctcgaggag cagacacctg aagagcctgg atctcacgga caatgccctg 3060
ggtgacggtg gggttgctgc actgtgcgag ggactgaagc aaaagaacag tgttctgacg 3120
agactcgggt tgaaggcatg tggactgact tctgattgct gtgaggcact ctccttggcc 3180
ctttcctgca accggcatct gaccagtcta aacctggtgc agaataactt cagtcccaaa 3240
ggaatgatga agctgtgttc ggcctttgcc tgtcccacgt ctaacttaca gataattggg 3300
ctgtggaaat ggcagtaccc tgtgcaaata aggaagctgc tggaggaagt gcagctactc 3360
aagccccgag tcgtaattga cggtagttgg cattcttttg atgaagatga ccggtactgg 3420
tggaaaaact gaagatacgg aaacctgccc cactcacacc catctgatgg aggaacttta 3480
aacgctgttt tctcagagca agctatgcac ctgggagttc cttctcaaag atggagaatg 3540
atttctgatt ctcacaaagc cctcaatggt agtgattctt ctgtgttcac tctacgttgg 3600
ttactggatt tgaaggctag agaccttcaa gtcataggac tcagtatctg tgaaatgtcc 3660
gtcatatctc agagcatata gagggaatta aataaacaca aagcatttgg aaaagttgtc 3720
aagtggtttt cttaactagt ggagatatgg tttaggagca gagaggttgg gaggacctag 3780
atcttcaaaa gaagcccctg aatttgggta ccacaactag tggtggtttt 3830

<210> 4
<211> 1143
<212> PRT
<213> Homo sapiens

<400> 4
Met Glu Gly Asp Lys Ser Leu Thr Phe Ser Ser Tyr Gly Leu Gln Trp
1               5                   10                  15

Cys Leu Tyr Glu Leu Asp Lys Glu Glu Phe Gln Thr Phe Lys Glu Leu
            20                  25                  30

Leu Lys Lys Lys Ser Ser Glu Ser Thr Thr Cys Ser Ile Pro Gln Phe
        35                  40                  45

Glu Ile Glu Asn Ala Asn Val Glu Cys Leu Ala Leu Leu Leu His Glu
    50                  55                  60

Tyr Tyr Gly Ala Ser Leu Ala Trp Ala Thr Ser Ile Ser Ile Phe Glu
65                  70                  75                  80

Asn Met Asn Leu Arg Thr Leu Ser Glu Lys Ala Arg Asp Asp Met Lys
                85                  90                  95

Asn Ser Pro Glu Asp Pro Glu Ala Thr Met Thr Asp Gln Gly Pro Ser
            100                 105                 110

Lys Glu Lys Val Pro Glu Asn Lys Tyr Gly Met Thr Lys Leu Ile Leu
        115                 120                 125

Gly Val Ser Asp Ile Ser Asp Ser Asn Asn Lys His Lys Tyr Val Gly
    130                 135                 140

Ile His Ser Ser Phe Ala Glu Ile Ser Gln Ala Met Glu Gln Glu Gly
145                 150                 155                 160

Ala Thr Ala Ala Glu Thr Glu Glu Gln Gly His Gly Gly Asp Thr Trp
                165                 170                 175

Asp Tyr Lys Ser His Val Met Thr Lys Phe Ala Glu Glu Glu Asp Val
            180                 185                 190

Arg Arg Ser Phe Glu Asn Thr Ala Ala Asp Trp Pro Glu Met Gln Thr
        195                 200                 205

Leu Ala Gly Ala Phe Asp Ser Asp Arg Trp Gly Phe Arg Pro Arg Thr
    210                 215                 220

Val Val Leu His Gly Lys Ser Gly Ile Gly Lys Ser Ala Leu Ala Arg
225                 230                 235                 240

Arg Ile Val Leu Cys Trp Ala Gln Gly Gly Leu Tyr Gln Gly Met Phe
                245                 250                 255

Ser Tyr Val Phe Phe Leu Pro Val Arg Glu Met Gln Arg Lys Lys Glu

            260                 265                 270

Ser Ser Val Thr Glu Phe Ile Ser Arg Glu Trp Pro Asp Ser Gln Ala
        275                 280                 285

Pro Val Thr Glu Ile Met Ser Arg Pro Glu Arg Leu Leu Phe Ile Ile
    290                 295                 300

Asp Gly Phe Asp Asp Leu Gly Ser Val Leu Asn Asn Asp Thr Lys Leu
305                 310                 315                 320

Cys Lys Asp Trp Ala Glu Lys Gln Pro Pro Phe Thr Leu Ile Arg Ser
                325                 330                 335

Leu Leu Arg Lys Val Leu Leu Pro Glu Ser Phe Leu Ile Val Thr Val
            340                 345                 350

Arg Asp Val Gly Thr Glu Lys Leu Lys Ser Glu Val Val Ser Pro Arg
        355                 360                 365

Tyr Leu Leu Val Arg Gly Ile Ser Gly Glu Gln Arg Ile His Leu Leu
    370                 375                 380

Leu Glu Arg Gly Ile Gly Glu His Gln Lys Thr Gln Gly Leu Arg Ala
385                 390                 395                 400

Ile Met Asn Asn Arg Glu Leu Leu Asp Gln Cys Gln Val Pro Ala Val
                405                 410                 415

Gly Ser Leu Ile Cys Val Ala Leu Gln Leu Gln Asp Val Val Gly Glu
            420                 425                 430

Ser Val Ala Pro Phe Asn Gln Thr Leu Thr Gly Leu His Ala Ala Phe
        435                 440                 445

Val Phe His Gln Leu Thr Pro Arg Gly Val Val Arg Arg Cys Leu Asn
    450                 455                 460

Leu Glu Glu Arg Val Val Leu Lys Arg Phe Cys Arg Met Ala Val Glu
465                 470                 475                 480

Gly Val Trp Asn Arg Lys Ser Val Phe Asp Gly Asp Asp Leu Met Val
                485                 490                 495

Gln Gly Leu Gly Glu Ser Glu Leu Arg Ala Leu Phe His Met Asn Ile
            500                 505                 510

Leu Leu Pro Asp Ser His Cys Glu Glu Tyr Tyr Thr Phe Phe His Leu
        515                 520                 525

Ser Leu Gln Asp Phe Cys Ala Ala Leu Tyr Tyr Val Leu Glu Gly Leu
    530                 535                 540

Glu Ile Glu Pro Ala Leu Cys Pro Leu Tyr Val Glu Lys Thr Lys Arg
545                 550                 555                 560

Ser Met Glu Leu Lys Gln Ala Gly Phe His Ile His Ser Leu Trp Met
                565                 570                 575

Lys Arg Phe Leu Phe Gly Leu Val Ser Glu Asp Val Arg Arg Pro Leu
            580                 585                 590

Glu Val Leu Leu Gly Cys Pro Val Pro Leu Gly Val Lys Gln Lys Leu
        595                 600                 605

Leu His Trp Val Ser Leu Leu Gly Gln Gln Pro Asn Ala Thr Thr Pro
    610                 615                 620

Gly Asp Thr Leu Asp Ala Phe His Cys Leu Phe Glu Thr Gln Asp Lys
625                 630                 635                 640

Glu Phe Val Arg Leu Ala Leu Asn Ser Phe Gln Glu Val Trp Leu Pro
                645                 650                 655

Ile Asn Gln Asn Leu Asp Leu Ile Ala Ser Ser Phe Cys Leu Gln His
            660                 665                 670

Cys Pro Tyr Leu Arg Lys Ile Arg Val Asp Val Lys Gly Ile Phe Pro
        675                 680                 685

Arg Asp Glu Ser Ala Glu Ala Cys Pro Val Val Pro Leu Trp Met Arg
    690                 695                 700

Asp Lys Thr Leu Ile Glu Glu Gln Trp Glu Asp Phe Cys Ser Met Leu
705                 710                 715                 720

Gly Thr His Pro His Leu Arg Gln Leu Asp Leu Gly Ser Ser Ile Leu
                725                 730                 735

Thr Glu Arg Ala Met Lys Thr Leu Cys Ala Lys Leu Arg His Pro Thr
            740                 745                 750

Cys Lys Ile Gln Thr Leu Met Phe Arg Asn Ala Gln Ile Thr Pro Gly
        755                 760                 765

Val Gln His Leu Trp Arg Ile Val Met Ala Asn Arg Asn Leu Arg Ser
    770                 775                 780

Leu Asn Leu Gly Gly Thr His Leu Lys Glu Glu Asp Val Arg Met Ala
785                 790                 795                 800

Cys Glu Ala Leu Lys His Pro Lys Cys Leu Leu Glu Ser Leu Arg Leu
                805                 810                 815

Asp Cys Cys Gly Leu Thr His Ala Cys Tyr Leu Lys Ile Ser Gln Ile
            820                 825                 830

Leu Thr Thr Ser Pro Ser Leu Lys Ser Leu Ser Leu Ala Gly Asn Lys
        835                 840                 845

Val Thr Asp Gln Gly Val Met Pro Leu Ser Asp Ala Leu Arg Val Ser
    850                 855                 860

Gln Cys Ala Leu Gln Lys Leu Ile Leu Glu Asp Cys Gly Ile Thr Ala
865                 870                 875                 880

Thr Gly Cys Gln Ser Leu Ala Ser Ala Leu Val Ser Asn Arg Ser Leu
                885                 890                 895

Thr His Leu Cys Leu Ser Asn Asn Ser Leu Gly Asn Glu Gly Val Asn

900                 905                 910

Leu Leu Cys Arg Ser Met Arg Leu Pro His Cys Ser Leu Gln Arg Leu
        915                 920                 925

Met Leu Asn Gln Cys His Leu Asp Thr Ala Gly Cys Gly Phe Leu Ala
    930                 935                 940

Leu Ala Leu Met Gly Asn Ser Trp Leu Thr His Leu Ser Leu Ser Met
945                 950                 955                 960

Asn Pro Val Glu Asp Asn Gly Val Lys Leu Leu Cys Glu Val Met Arg
                965                 970                 975

Glu Pro Ser Cys His Leu Gln Asp Leu Glu Leu Val Lys Cys His Leu
            980                 985                 990

Thr Ala Ala Cys Cys Glu Ser Leu Ser Cys Val Ile Ser Arg Ser Arg
        995                 1000                1005

His Leu Lys Ser Leu Asp Leu Thr Asp Asn Ala Leu Gly Asp Gly Gly
    1010                1015                1020

Val Ala Ala Leu Cys Glu Gly Leu Lys Gln Lys Asn Ser Val Leu Thr
1025                1030                1035                1040

Arg Leu Gly Leu Lys Ala Cys Gly Leu Thr Ser Asp Cys Cys Glu Ala
                1045                1050                1055

Leu Ser Leu Ala Leu Ser Cys Asn Arg His Leu Thr Ser Leu Asn Leu
            1060                1065                1070

Val Gln Asn Asn Phe Ser Pro Lys Gly Met Met Lys Leu Cys Ser Ala
        1075                1080                1085

Phe Ala Cys Pro Thr Ser Asn Leu Gln Ile Ile Gly Leu Trp Lys Trp
    1090                1095                1100

Gln Tyr Pro Val Gln Ile Arg Lys Leu Leu Glu Glu Val Gln Leu Leu
1105                1110                1115                1120

Lys Pro Arg Val Val Ile Asp Gly Ser Trp His Ser Phe Asp Glu Asp
                1125                1130                1135

Asp Arg Tyr Trp Trp Lys Asn
            1140